# EUROPEAN PATENT SPECIFICATION

(11) **EP 2 524 688 B1**
(45) Date of publication and mention of the grant of the patent: **01.05.2013**
(21) Application number: 11003866.8
(22) Date of filing: 11.05.2011
(51) Int. Cl.: A61K 9/16, A61K 9/20, A61K 9/28, A61K 31/495

(54) **Composition for modified release comprising ranolazine**
Zusammensetzung zur modifizierten Freisetzung mit Ranolazin
Composition de libération modifiée comportant de la ranolazine

(43) Date of publication of application: 21.11.2012
(73) Proprietor: ratiopharm GmbH, 89079 Ulm (DE)
(72) Inventor: Sahin, Hulya, Montreal H1E 2N2, QC (CA); Weber, Birgit, Dr., 63739 Aschaffenburg (DE)
(74) Representative: TER MEER - STEINMEISTER & PARTNER GbR

(56) References cited:
- EP-A2- 1 027 887
- WO-A1-2006/099244
- WO-A2-01/60348
- US-A1- 2008 312 247
- WILLIAM CURATOLO ET AL: "Utility of Hydroxypropylmethylcellulose Acetate Succinate (HPMCAS) for Initiation and Maintenance of Drug Supersaturation in the GI Milieu", PHARMACEUTICAL RESEARCH, KLUWER ACADEMIC PUBLISHERS-PLENUM PUBLISHERS, NL, vol. 26, no. 6, 10 March 2009 (2009-03-10) , pages 1419-1431, XP019686188, ISSN: 1573-904X, DOI: 10.1007/S11095-009-9852-Z

## Description

### Background of the invention

The invention essentially relates to a pharmaceutical composition comprising ranolazine and HPMC-AS, suitable for modified release, a process of preparing such a pharmaceutical composition and the use of HPMC-AS for preparing a pharmaceutical composition for treatment of Angina and insufficient blood supply to the heart.

Ranolazine, (RS)- N-(2,6-Dimethylphenyl)-2-[4-[2-hydroxy-3-(2-methoxyphenoxy)propyl]piperazin-1-yl]acetamide, is reported to have the chemical formula C₂₄H₃₃N₃O₄ and the following chemical structure (I):

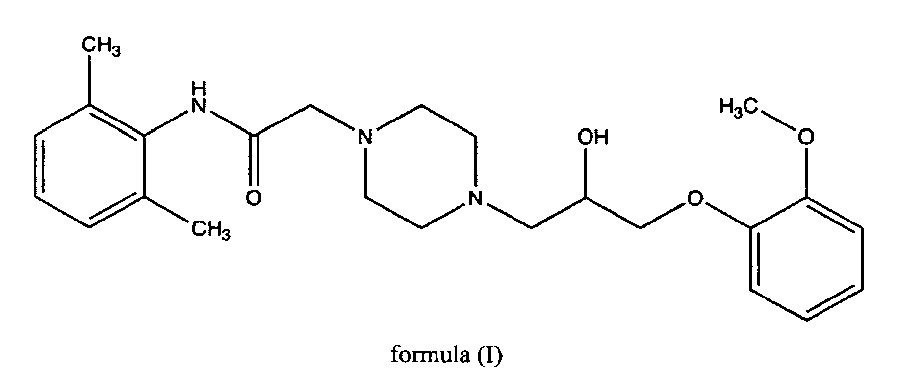

In WO 2006/074398 A2 it is mentioned that ranolazine can for example be used in the treatment of many disease states, including heart failure, arrhythmia, angina, diabetes, myocardial infarction and intermittent claudication.

The presently preferred route of administration is oral; therefore, a typical dosage form is a tablet. Former tablets were suffering from relatively high solubility of ranolazine at the pH occurring in the stomach and its short plasma half-life. These result in rapid drug absorption and clearance, while causing large fluctuations in plasma concentration. To overcome these problems EP 1 109 558 B1 discloses sustained-release ranolazine compositions to be administered to a patient once or twice a day while maintaining the plasma ranolazine close to the minimal effective levels without peak fluctuations. This is achieved by using a sustained-release pharmaceutical dosage essentially comprising at least 50% by weight ranolazine and at least one pH-dependent binder.

The above-mentioned WO 2006/074398 A2 also discloses pharmaceutical compositions according to the sustained release of the active pharmaceutical ingredient. One of the compositions comprises 35-49 % of ranolazine, a pH-dependent binder, a pH-independent binder and one or more pharmaceutically acceptable excipients.

As shown in the description and in the examples of both WO 2006/074398 A2 and EP 1 109 558 B1, the use of either a partially neutralized methacrylic acid copolymer or a phthalat of hydroxypropyl cellulose, hydroxypropyl methylcellulose, cellulose acetate, polyvinyl acetate or polyvinyl pyrrolidone as a pH-dependent binder is essentially taught.

WO 01/60 348 discloses sustained release compositions comprising ranolazine and HPMC.

It turned out that the stability of ranolazine in a sustained-release ranolazine composition comprising one or more of the above-mentioned pH-dependent binder(s) is not ideal, since, especially under stress conditions, significant degradation of the ranolazine is noticed. The degradation of ranolazine can lead to an undesirable low content-uniformity, resulting in unpredictable and unreproducible dissolution rates and bioavailability.

Hence, there is a need for pharmaceutical compositions and processes to manufacture these pharmaceutical compositions, which do not suffer from the above-mentioned drawbacks. Preferably, pharmaceutical compositions should be provided having improved properties like stability, content-uniformity, well-defined, predictable and reproducible dissolution rates and bioavailability. Such a pharmaceutical composition should be producible in a large scale in an economically beneficial way.

### Summary of the invention

According to the present invention the above objectives preferably are achieved by specific ranolazine compositions comprising a specific non-erodible substance as excipient. Furthermore, the above drawbacks can be overcome by a process for producing said compositions.

Thus, the subject of the present invention is a pharmaceutical composition comprising
(a) ranolazine, and
(b) hypromellose-acetate-succinate (HPMC-AS).

It was found that the pharmaceutical composition of the present invention can be surprisingly stable over a long period, even under common stress conditions. The administration of the pharmaceutical composition of the present invention results in advantageously little blood level fluctuation, which means that periods of transient therapeutic overdose, followed by a period of therapeutic underdosing can be avoided.

Moreover, the pharmaceutical composition of the present invention is released in the gastrointestinal tract of the patient but not in the stomach in order to avoid "nervous stomach" or nausea.

A further subject of the present invention is a process for manufacturing the pharmaceutical composition of the present invention, preferably in form of a modified release tablet. The process for manufacturing a pharmaceutical composition comprising the steps of
(1-I) mixing components (a), (b) and optionally a pore-forming substance (c),
(1-II) adding water to the mixture,
(1-III) wet-granulating the mixture,
(1-IV) drying and optionally milling the granulates,
(1-V) optionally adding further excipients (d),
(1-VI) optionally compressing the composition into tablets, and
(1-VII) optionally film-coating the product.

Another subject of the present invention is the use of HPMC-AS for preparing a pharmaceutical composition for the treatment of angina pectoris and insufficient blood supply of the heart.

### Detailed Description of the invention

In the following, explanations regarding the pharmaceutical composition of the present invention are given. However, these explanations can also apply to the processes for manufacturing the pharmaceutical dosage form, such as the modified-release tablet of the present invention, and to the use of the present invention.

Within the present application, the term "modified release" can generally be used as defmed by the USP. Preferably, modified-release dosage forms can be those whose drug-release characteristics accomplish therapeutic or convenience objectives not offered by immediate release forms. Generally, immediate-release (IR) forms are reported to release at least 70 % of the drug within 1 hour or less. The term "modified release" can comprise delayed release, prolonged release, sustained release, extended release and/or controlled release.

Delayed release is reported to indicate that the drug (= ranolazine) is not released immediately after administration but at a later time, preferably less than 10 % are released within two hours after administration.

Prolonged release is reported to indicate that the drug (= ranolazine) is provided for absorption over a longer period of time than in the case of IR forms, preferably for about 2 to 24 hours, in particular for 3 to 12 hours.

Sustained release is reported to indicate an initial release of the drug (= ranolazine), sufficient to provide a therapeutic dose soon after administration, preferably within two hours after administration, and then a gradual release after an extended period of time, preferably for about 3 to 18 hours, in particular for 4 to 8 hours.

Extended release is reported to indicate a slow drug (= ranolazine) release, so that plasma concentrations are maintained at a therapeutic level for a time period of between 6 and 36 hours, preferably between 8 and 24 hours.

Controlled-release dosage forms are reported to release the drug (= ranolazine) at a constant rate and provide plasma concentrations that remain essentially invariant with time.

In a preferred embodiment the pharmaceutical composition of the present invention can be a pharmaceutical composition showing sustained release properties.

Within this application the release profiles are generally determined according to USP 31-NF26 release method, apparatus 2 (paddles) with 50 rpm. The measurements are carried out at 37 ± 0.5°C, in 0.1 HCl (pH 1.2).

In a preferred embodiment, the pharmaceutical composition of the present invention can be a solid pharmaceutical composition.

The term ranolazine (component (a)) as used in the present invention is reported to relate to the compound as shown in formula I (free base) or to its acid form or its basic form. This means that "ranolazine" as used in the present invention also can relate to its pharmaceutically acceptable salts, preferably pharmaceutically acceptable acid addition salts, for example as described in WO 02/096909 A1. The acids, which can be used to prepare the pharmaceutically acceptable acid addition salts, are preferably those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [1,1'-methylene-bis-(2-hydroxy-3-naphthoate)] salts.

The term "ranolazine" can also relate to stereospecific base addition salts of formula (I). The chemical bases that may be used as reagents to prepare pharmaceutically acceptable base salts of those compounds of formula (I) that are acidic in nature are those that form non-toxic base salts with such compounds. Such non-toxic base salts can include, but are not limited to, those derived from such pharmacologically acceptable cations, such as alkali metal cations (e.g. potassium and sodium) and alkaline earth metal cations (e.g. calcium and magnesium), ammonium or water-soluble amine addition salts, such as N-methyl glucamine (meglumine), and the lower alkanol ammonium and other base salts of pharmaceutically acceptable organic amines.

In preferred embodiment of the present invention, ranolazine as the active ingredient (component (a)) can be provided in amorphous form, in crystalline form or as a mixture of both forms.

In a preferred embodiment the pharmaceutical composition of the present invention can comprise less than 50% by weight, preferably 35 to 49% by weight ranolazine, based upon the total weight of the pharmaceutical composition and based on the weight of ranolazine in form of the free base, i.e. as shown in formula (I) above.

In a preferred embodiment the pharmaceutical composition of the present invention can comprise 50 to 800 mg, more preferably 150 to 600 mg, still more preferably 250 to 500 mg, in particular 300 to 450 mg ranolazine, based upon the total weight of the pharmaceutical composition and based on the weight of ranolazine in form of the free base, i.e. as shown in formula (I) above.

The pharmaceutical composition of the present invention contains hypromellose-acetate-succinate (b), which is referred to in the following as HPMC-AS. HPMC-AS can further be referred to as hydroxypropylmethylcellulose-acetate-succinate. HPMC-AS can be regarded as non-erodible material. HPMC-AS can preferably be suitable as release-controlling agent. HPMC-AS is reported to be a partially esterified derivate of HPMC and to have the following chemical structure (II), 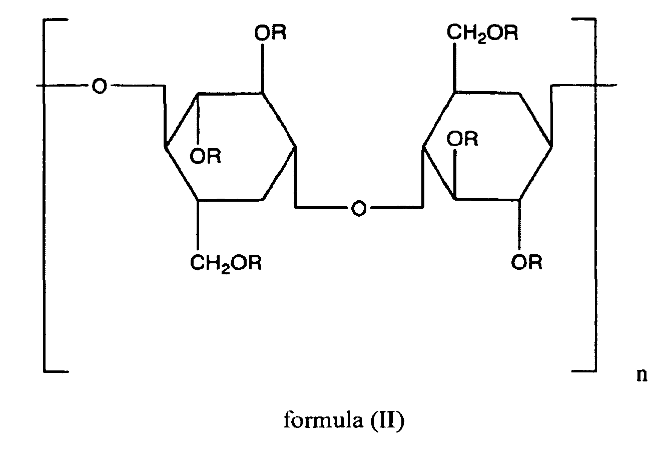 wherein R is selected form H, CH₃, CH₂CH(CH₃)OH, COCH₃, COCH₂CH₂COOH, CH₂CH(CH₃)OCOCH₃ and CH₂CH(CH₃)OCOCH₂CH₂COOH. The ratio of acetyl to succinoyl substitution can be from 8:15 to 12:7.

In a preferred embodiment the HPMC-AS can be described as providing a scaffold (matrix) for embedding the active ingredient and to form a physical barrier, which hinders the active ingredient from being released immediately from the dosage form. Thus, the non-erodible material may have the effect that the active ingredient can be released from the scaffold in continuous manner. Release of the drug from the matrix can be dissolution-controlled as well as diffusion-controlled mechanisms. In this first embodiment the non-erodible material can function as matrix-forming material.

Generally, the pharmaceutical composition of the present invention can further comprise HPMC-AS (b) as non-erodible material. Usually, non-erodible materials are materials, which are able to provide modified-release properties, preferably due to their limited solubility, more preferably due to their limited solubility in aqueous conditions at pH 5.0. Preferably, the non-erodible material (b) can have a water solubility of less than 33 mg/l at a temperature of 25 °C at a pH of 5.0, more preferably of less than 22 mg/l, still more preferably of less than 11 mg/l, especially from 0.01 to 5 mg/l. The water solubility can be determined according to the column elution method of the Dangerous Substances Directive (67/548/EEC), Annex V, Chapter A6. The pH value can be determined according to Ph. Eur. 6.0, Chapter 2.2.3. The pH value of the aqueous medium usually can be achieved by addition of HCl (or NaOH), if necessary.

The solubility of the non-erodible material (HPMC-AS) preferably can be pH-dependent. It is preferred that the non-erodible material may have a solubility in water at 25 °C at a pH of 7.0 of more than 25 g/l, more preferably of more than 50 g/l, still more preferably of more than to 500 g/l.

In a preferred embodiment the non-erodible material (HPMC-AS) can have a weight average molecular weight ranging from 5000 to 300000 g/mol, preferably from more than 8000 to 100000 g/mol, more preferably from more than 10000 to 50000 g/mol. Furthermore, a 2 % w/w solution of HPMC-AS in water at pH 7.0 preferably can have a viscosity of more than 2 mPas, more preferably of more than 5 mPas, particularly more than 8 mPas and up to 850 mPas when measured at 25 °C. The viscosity can be determined according to Ph. Eur. 6.0, Chapter 2.2.10. In the above definition the term "solution" may also refer to a partial solution (in case that the polymer does not dissolve completely in the solution). The weight average molecular weight can preferably be determined by gel electrophoresis.

In a preferred embodiment the HPMC-AS (b) can be contained in the pharmaceutical composition of the present invention in an amount of 5 to 50 % by weight, preferably of 10 to 40 % by weight, more preferably of 15 to 35 % by weight, based upon the total weight of the pharmaceutical composition. If too little HPMC-AS is used, the compositions may break up during the passage down the gastrointestinal tract and this, in turn, may lead to a premature release of a large portion of the drug. If too much HPMC-AS is used, there is a risk that some of the drug will not be sufficiently released from the tablet.

The pharmaceutical composition of the invention can further preferably comprise a pore-forming substance (c). The term "channeling agent" can be used synonymously in the art for the pore-forming substance (c) of the present invention. Since the pore-forming substance can be generally soluble in the gastrointestinal tract and leaches out of the pharmaceutical composition, the pore-forming substance can be described has having the effect of forming pores, such as small holes within the tablet, through which the active ingredient can be released from the tablet matrix in a controlled manner.

The pore-forming substance (c) of the present invention can preferably have a water-solubility from 30 g/l to 3000 g/l at a temperature of 25 °C and more preferred from 150 mg/l to 300 g/l. The water-solubility can be determined according to the column elution method of the Dangerous Substances Directive (67/548/EEC), Annex V, Chapter A6.

In an embodiment of the present invention the pore-forming substance (c) can be selected from organic substances, in particular from organic substances being solid at 30 °C and having the above-mentioned water solubility. Sugar alcohols like mannitol, sorbitol, xylitol, isomalt, and mono or disaccharides, like lactose, can be preferred as pore-forming substances (c). Especially mannitol can be preferred as pore-forming substance (c).

Preferably the pore-forming substance (c) can be a particulate solid. Further preferably, the average particle diameter (D₅₀) of the pore-forming substance (c) can be between 10 and 200 µm, more preferably between 20 and 175 µm, still more preferably between 30 and 100 µm and most preferably between 35 and 65 ¨µm.

The expression "average particle diameter" refers in the context of this invention to the D₅₀ value of the volume-average particle diameter determined by means of laser diffractometry. In particular, a Malvern Instruments Mastersizer 2000 was used to determine the diameter (wet measurement with ultrasound 60 sec., 2,000 rpm, preferably dispersion in liquid paraffin, the evaluation being per-formed according to the Fraunhofer model). The average particle diameter, which is also referred to as the D₅₀ value of the integral volume distribution, is defined in the context of this invention as the particle diameter at which 50 % by volume of the par-ticles have a smaller diameter than the diameter which corresponds to the D₅₀ value. Similarly, 50 % by volume of the particles than have a larger diameter than the D₅₀ value.

The pore-forming substance (c) can be contained in the pharmaceutical composition in an amount of 5 to 50 % by weight, preferably of 10 to 40 % by weight, more preferably of 15 to 35 % by weight, based upon the total weight of the pharmaceutical composition.

The pharmaceutical composition of the present invention can further comprise at least one excipient (d), selected from lubricants (d1), disintegrants (d2), surfactants (d3), glidants (d4), anti-sticking agents (d5), plasticizers (d6) and mixtures thereof.

The function of lubricants (d1) is reported to ensure that tablet formation and ejection can occur with low friction between the solid and the die wall. The lubricant is preferably a stearate or fatty acid, more preferably an earth alkali metal stearate, such as magnesium stearate. The lubricant can be present in an amount of 0 to 2 % by weight, preferably of about 0.1 to 1.0 % by weight, based on the total weight of the pharmaceutical composition. Lubricants generally can increase the powder flowability.

In a preferred embodiment of this invention, a lubricant (d1) may be used. In a preferred embodiment of the present invention magnesium stearate can be used as lubricant (d1).

Disintegrants (d2) are reported to be compounds, which enhance the ability of the dosage form, preferably the ability of the tablet, when in contact with a liquid, preferably water, to break into smaller fragments. Disintegrants (d2) can preferably be sodium carboxymethyl starch, cross-linked polyvinylpyrrolidone (crospovidone), sodium carboxymethyl glycolate (for example Explotab^{®}), swelling polysaccharide, for example soy polysaccharide, carrageenan, agar, pectin, starch and derivates thereof, protein, for example formaldehyde-casein, sodium bicarbonate or mixtures thereof. Disintegrants can be used in an amount of 0 to 20 % by weight, preferably of 1 to 10 % by weight, based on the total weight of the pharmaceutical composition.

Surfactants (d3) can be regarded as substances lowering the interfacial tension between two phases, thus enabling or supporting the formation of dispersions or working as solubilizer. Common surfactants are alkylsulfates (for example sodium lauryl sulfate), alkyltrimethylammonium salts, alcohol ethoxylates and the like. Surfactants can be used in an amount of 0.05 to 2 % by weight, preferably of 0.1 to 1.5 % by weight, based on the total weight of the pharmaceutical composition.

Glidants (d4) are reported to be substances used to improve the flowability. Traditionally, talc was used as glidant but it is nowadays nearly fully replaced by colloidal silica (for example Aerosil^{®}). Preferably, the glidant (d4) can be present in an amount of up to 3 % by weight, based on the total weight of the pharmaceutical composition. Preferably, the silica has a specific surface area of 50 to 400 m²/g, measured by gas adsorption according to Ph. Eur. 6.0, Chapter 2.9.26, multipoint method, volumetric determination.

In a preferred embodiment of the present invention silicon dioxide can be used as a glidant (d4).

Generally, anti-sticking agents (d5) are reported to be substances to prevent the adhesion of the tableting mass to the compression mould. Further anti-sticking agents may increase the brightness of a tablet. The anti-sticking agent can be for example talcum, magnesium stearate, paraffin and the like. Further, the anti sticking agent (d5) may be present in amounts of 0 to 5 % by weight, based on the total weight of the pharmaceutical composition.

Plasticizers (d6) usually are reported to be compounds capable of lowering the glass transition temperature (T_{g}) of a non-erodible material, preferably of lowering T_{g} from 1 to 50 °C. Plasticizers (c8) can be low molecular weight compounds (having a molecular weight of 50 to 500 g/mol) and can comprise at least one hydrophilic group. Examples of suitable plasticizers are dibutyl sebacetate (DBS), Myvacet^{®} (acetylated monoglycerides), triacetin (GTA), citric acid esters, like acetyltriethyl citrate (ATEC) or triethyl citrate (TEC), propylene glycol, dibutyl phthalate, diethyl phthalate, or mixtures thereof.

Regarding the above-mentioned pharmaceutically acceptable excipients, the application generally refers to "Lexikon der Hilfsstoffe für Pharmazie, Kosmetik und angrenzende Gebiete", edited by H. P. Fiedler, 5th Edition, Editio Cantor Verlag, Aulendorf and earlier editions, and "Handbook of Pharmaceutical Excipients", third edition, edited by Arthur H. Kibbe, American Pharmaceutical Association, Washington, USA, and Pharmaceutical Press, London.

In this regard it is generally noted that due to the nature of pharmaceutical excipients, it cannot be excluded that a certain compound meets the requirements of more than one of the components (d1) to (d6). However, to enable an unambiguous distinction, it is preferred in the present application that one and the same pharmaceutical compound is only used as one of the compounds (d1) to (d6). For example, if magnesium stearate is used as lubricant (d1) it is not additionally used as anti-sticking agent (d5), even though magnesium stearate also exhibits a certain anti-sticking effect.

Another preferred embodiment the pharmaceutical composition of the present invention is in the form of a tablet, preferably a matrix tablet.

In a further preferred embodiment of the present invention the hardness of the tablet is from 40 to 350 N, more preferred from 50 to 325 N, still more preferred from 75 to 300 N, in particular from 85 to 275 N, wherein the hardness is measured according to Ph. Eur. 6.0, Chapter 2.9.8.

Surprisingly, it was found that the hardness of a tablet according to the present invention results in a superior dissolution profile of the drug.

In addition, the resulting tablets can preferably have a friability of less than 5 %, particularly preferably less than 2 %, especially less than 1 %. The friability is determined in accordance with Ph. Eur. 6.0, Chapter 2.9.7. The friability generally refers to the tablet without coating.

Finally, the tablets of the invention preferably can have a "content uniformity" of 90 to 110 %, more preferably 95 to 105 %, especially 98 to 102 % of the average content. The "content uniformity" is determined in accordance with Ph. Eur. 6.0, Chapter 2.9.6.

In another preferred embodiment the tablet according the present invention is film-coated with a coating (e).

In the present invention, the following three types of film-coatings are possible:
- film-coating without effecting the release of the active ingredient (preferred),
- gastric juice resistant film-coatings,
- retard coatings.

Film-coatings that do not effect the release of the active ingredient are preferred. In gastric juice resistant coatings the solubility depends on the pH of the surrounding. Retard coatings are usually non-soluble (preferably having a water-solubility at 25 °C of less than 10 mg/ml).

Generally, film-coatings can be prepared by using cellulose derivatives, poly(meth)-acrylate, polyvinylpyrrolidone, polyvinyl acetate phthalate, and/or shellac or natural rubbers such as carrageenan.

Preferred examples of coatings (e), which do not effect the release of the active ingredient can be those including poly(meth)acrylate, methylcellulose (MC), hydroxypropyl methylcellulose (HPMC), hydroxypropyl cellulose (HPC), hydroxyethyl cellulose (HEC), polyvinylpyrrolidone (PVP) and mixtures thereof. These polymers can have a median molecular weight of 10,000 to 150,000 g/mol.

Examples of gastric juice resistant coatings can comprise cellulose acetate phthalate (CAP), hydroxypropyl methylcellulose phthalate (HPMCP) and polyvinyl acetate phthalate (PVAP). Examples of retard coatings can comprise ethyl cellulose (EC, commercially available e.g. as Surelease^{®}) and poly(meth)acrylate (commercially available e.g. as Eudragit^{®} RL or RS and L/S).

The coating (e) can be free of active ingredient. However, it is also possible that the coating can contain active ingredient (ranolazine). In such a case, that amount of active ingredient would function as an initial dose. In such a case the coating (e) preferably can comprise 1 to 45 wt.%, preferably 5 to 35 wt.%, most preferably 10 to 30 wt.% of ranolazine, based on the total amount of ranolazine contained in the tablet. In this application, the coating preferably is a coating, which does not effect the release of ranolazine.

In case the film coating does not contain ranolazine (which is preferred), it can have a thickness of 2 µm to 100 µm, preferably from 20 to 60 µm. In case of a coating containing ranolazine, the thickness of the coating is usually 10 µm to 2 µm, preferably from 50 to 500 µm.

The preferred coating (e) may comprise
(e1) film-forming agent;
(e2) lubricant;
(e3) surfactant;
(e4) glidant;
(e5) pigment; and/or
(e6) water.

Since in a preferred embodiment the solubility of the coating should be based on a pH-dependent binder, a polymeth(acrylate) polymer can be preferably contained as a film-forming agent (e1). Such a preferred polymeth(acrylate) polymer can dissolve readily in acidic media of pH < 5 via salt formation and, therefore, does not affect further tablet disintegration and release behaviour. A preferred polymeth(acrylate) polymer (for example Eudragit^{®} E PO) can have the following chemical structure (III) 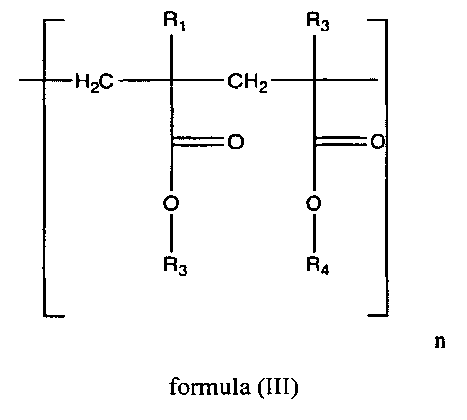 wherein R₁ and R₂ are CH₃, R₃ is (CH₂)₂N(CH₃)₂, and R₄ is CH₃ or C₄H₉. The molecular weight is from 20000 to 300000 g/mol, more preferred from 30000 to 150000 g/mol and particularly form 35000 to 80000 g/mol.

The preferred coating (e) according to an embodiment of the present invention can comprise, along with the film-forming agent (e1), e.g. stearic acid as lubricant (e2) for plasticizing and dissolving the polymer, sodium lauryl sulfate as a surfactant (e3) for wetting and dispersing, talc as glidant (e4), iron oxide yellow and/or titane oxide as pigment(s) (e5) and optionally purified water.

In a further embodiment of the subject invention the pharmaceutical composition is a modified release pharmaceutical composition.

Accordingly, in a further preferred embodiment the modified release pharmaceutical composition can relate to a tablet in which 1 to 45 wt.%, preferably 5 to 35 wt.%, most preferably 10 to 30 wt.% of the total amount of the ranolazine contained in the tablet can be present as initial doses having immediate release, and 55 to 99 wt.%, preferably 65 to 95 wt.%, most preferably 70 to 90 wt.% of the active ingredient can be present in the tablet as a modified-release composition.

An embodiment of a further subject of the invention relates to a process for manufacturing the above-mentioned-pharmaceutical composition comprising the steps of
(1-I) providing components (a), (b) and optionally (c) and/or (d),
(1-II) adding water and/or a solvent and optionally excipient (d),
(1-III) wet-granulating the resulting mixture,
(1-IV) drying and optionally milling the granulates,
(1-V) optionally adding excipient (d), and
(1-VI) optionally compressing the composition into tablets,
(1-VII) optionally film-coating the tablets.

Preferred embodiments of the process of the present invention are described in more detail below.

In step (1-I) components (a), (b) optionally (c) are provided. They can be provided in micronized form. The step of micronization can preferably be carried out by milling, such as in an air jet mill. Preferably, the average particle size D₅₀ of ranolazine (a) is from 20 to 120 µm, and of component(s) (b) and/or (c) it is from 30 to 150 µm.

The average particle size D₅₀ is determined as described above.

Optionally, the ingredients (a), (b) and optionally (c) can be blended in order to provide a composition having a homogenous distribution of ranolazine (a) within the resulting blend, comprising (a), (b) and optionally (c). Blending can be carried out with conventional mixing devices, e.g. in a free-fall mixer like Turbula^{®} T10B (Bachofen AG, Switzerland). Blending can be carried out e.g. for 1 minute to 30 minutes, preferably for 2 minutes to less than 10 minutes.

Generally, step (1-II), i.e. "adding water and/or solvent" to the components (a), (b) and optionally (c) can be performed for forming a wetted granulation mass.

In step (1-II) of the above-mentioned process the solvent may be an organic solvent. Generally, suitable organic solvents might be selected form C₃-C₆ ketone, a C₅-C₉ aliphatic or aromatic hydrocarbon, optionally substituted for example with halogen, a C₃-C₆ ester, a C₂-C₆ alcohol, C₂-C₆ ether, DMAc, DMSO, NMP and mixtures thereof. In a preferred embodiment the solvent can be a C₂-C₆ alcohol, preferably ethanol.

In step (1-III), i.e. wet-granulating, the mixture of step (1-II) can be kneaded in a mixer, preferably in a fluid or intensive mixer or in a planetary compulsory mixer, and then brought into the desired particle form and size by sieving or by being passed through a perforated disc device. Herein the particles can be attached to each other, thereby giving larger particles. The attachments may occur through physical forces, preferably van der Waals forces. The attachment of particles preferably does not occur through chemical reactions.

The process conditions in the granulation step can preferably be selected such that the resulting granulates can have an average particle size D₅₀ of 50 to 750 µm, more preferably 150 to 400 µm.

The average particle size D₅₀ is determined as described above.

The obtained granulates from step (1-III) can preferably be dried and optionally milled in step (1-IV), giving dry and uniform granulates, which allow easy further processing.

Step (1-IV) of drying can preferably be carried out by removing the residual solvent. The removal of the solvent can be conducted under elevated temperature and/or under reduced pressure. In a preferred embodiment the solvent is removed at a temperature between 30 and 90 °C, preferably between 35 and 75 °C more preferably between 40 and 60 °C. The solvent can preferably be removed at a pressure from 0.01 to 900 mbar, preferably from 1 to 200 mbar, more preferably from 5 to 100 mbar, still more preferably from 10 to 50 mbar, in particular from 30 to 40 mbar. At laboratory scale the duration of step (1-IV) might range from 0.5 to 2.0 hours, preferably about 90 minutes. The removal of the solvent can be, for example, carried out in a vacuum rotary evaporator, for example a Büchi^{®} Rotavapor.

Step (1-V) can include the optional addition of at least one excipient (d), preferably selected from lubricants (d1), disintegrants (d2), surfactants (d3), glidants (d4), anti-sticking agents (d5), plasticizers (d6) and mixtures thereof. In a preferred embodiment magnesium stearate can be used as lubricant (d1) to reduce gripping and ejection force.

Step (1-VI) of optionally compressing the composition into tablets can be carried out on a rotary press, e.g. on a Fette^{®} (Fette GmbH, Germany) or a Riva^{®} piccola (Riva, Argentina. If a rotary press is applied, the main compression force can range from 1 to 50 kN, preferably 3 to 40 kN, more preferably 5 to 30 kN. The resulting tablets can preferably have a hardness of 40 to 350 N, more preferred 50 to 325 N, still more preferred of 75 to 300 N, in particular from 85 to 275 N, wherein the hardness can be measured according to Ph. Eur. 6.0, Chapter 2.9.8.

The tablets of the present invention can be film-coated in process step (1-VII). Said step (1-VII) can comprise optionally film-coating the tablet with a coating (e) comprising preferably (e1) a film-forming agent, (e2) a surfactant, (e3) a lubricant, (e4) a glidant, (e5) one or more pigments and (e6) purified water as described herein before.

The film-coating (e) can be applied preferably by spraying a coating solution onto the tablets and then drying the tablets. The coating solution may comprise the above-mentioned excipients, whereby a solid content of about 15% is preferred.

Further, as described before, the applied film coating (e) preferably should not affect the tablet disintegration and release behaviour. The thickness of the coating (e) ranges from 0.01 µm to 2 mm, preferably from 50 to 500 µm.

The coating process can be carried out in a continuous process in a pan coater or a fluid bed dryer. The coating process can preferably be carried out on a pan coater, for example on a Lödige LHC 25 (Lödige GmbH, Germany). If a pan coater is applied, the spray pressure can range from 0.8 to 2 bar, preferably from 1 to 1.5 bar. The product temperature can vary according to the applied polymer. The product temperature can be adjusted to 20 to 40 °C, preferably to 32 to 38 °C.

The release profiles of a non-coated tablet or a coated tablet, wherein the coating is free of the drug, can show a constant release as determined by method USP (paddle). Preferably, the slope of the initial drug release can be less than 0.6 to 0.8 % per minute.

Generally, it is noted that all comments made above with respect to the tablets (pharmaceutical composition) of the present invention also apply to the process of manufacturing such a tablet (pharmaceutical composition).

Consequently, further subjects of the present invention can be tablets obtainable by any of the processes as described above.

All explanations given above for the process of the present invention may also apply to the tablet of the present invention.

In a preferred embodiment, the pharmaceutical composition of the present invention can be suitable for administration once or twice per day, most preferably once per day. Alternatively, the pharmaceutical composition of the present invention can be administered every second day, thrice a week, twice a week or once a week.

The present invention is illustrated by the following examples.

### EXAMPLES

The following commercially available compounds were used in the examples below:
- Eudragit^{®} E PO (Evonik) :: copolymer of acrylic and methacrylic acid esters, containing tertiary ammonium groups
- Aerosil^{®}:: dispersed silicium dioxide

### Examples 1 to 3: Compositions containing ranolazine

### Example 1: Pharmaceutical composition 1

| | |
|---|---|
| Ranolazine | 375 mg (based on the free base) |
| HPMC-AS | 160 mg |
| Mannitol | 250 mg |
| Magnesium stearate | 12 mg |

Ranolazine, HPMC-AS and mannitol were sieved (1.25 mm mesh) into the pot of a Diosna^{®} P1-6 wet granulator and blended for 2 min. This pre-mixture was granulated, adding a suitable amount of water to gain a mixture having an easy-to-handle consistency. The wet granulate was sieved (2 mm mesh) and dried for 2 h at 40 °C in a cabinet drier. The dried granulate was sieved (1.25 mm mesh) and sieved (500 µm mesh) magnesium stearate was added. The resulting mixture was blended in a free fall mixer for 5 min.

### Example 2: Tablet made of the pharmaceutical composition 1

| | |
|---|---|
| Ranolazine | 375 mg (based on the free base) |
| HPMC-AS | 160 mg |
| Mannitol | 250 mg |
| Magnesium stearate | 12 mg |

Ranolazine, HPMC-AS and mannitol were sieved (1.25 mm mesh) into the pot of a Diosna^{®} P1-6 wet granulator and blended for 2 min. This pre-mixture was granulated, adding a suitable amount of water to gain a mixture having an easy-to-handle consistency. The wet granulate was sieved (2 mm mesh) and dried for 2 h at 40 °C in a cabinet drier. The dried granulate was sieved (1.25 mm mesh) and sieved (500 µm mesh) magnesium stearate was added. The resulting mixture was blended in a free fall mixer for 5 min and the final blend was compressed with 16 kN into tablets.

### Example 3: Film-coating of tablets according to Example 2

Tablets were formed according to Example 2.

**Tablet coating**

| | |
|---|---|
| Eudragit^{®} E PO | 2.8 mg |
| Stearic acid | 0.4 mg |
| Sodium lauryl sulfate | 0.3 mg |
| Talc | 1.4 mg |
| Ironoxide yellow | 0.7 mg |
| Titanium dioxide | 0.7 mg |
| Purified water | 33.6 mg |

An aqueous suspension of the Eudragit^{®} E PO with sodium lauryl sulfate as wetting and dispersing agent and stearic acid to plasticize the polymer was formed. Further talc as glidant and finally dyes for coloured coatings were added.

The coating process was carried out on a pan coater, for example on a Lödige LHC 25 (Lödige GmbH, Germany). The spray pressure ranges from 1 to 1.5 bar. The product temperature varies according to the applied polymer from 32 to 38 °C.

### Example: Stability data

In Table 1 assay and degradation products results for ranolazine per se, a pharmaceutical composition of the present invention, comprising ranolazine and HPMC-AS as well as a comparative pharmaceutical composition known from the prior art under stressed conditions are shown. Herein the stressed conditions are defined as storage over 5 weeks at 40 °C with 75 % RH and open cap.

**Table 1**

| | Assay (%) | Biggest impurity % (RRt) | Total degradation products (%) |
|---|---|---|---|
| Ranolazine/ HPMC-AS | 99.9 | 0.062 | 0.130 |
| | | (3.257) | |
| Ranolazine (raw material) | 99.6 | 0.066 | 0.155 |
| | | (3.229) | |
| Ranolazine/ Eudragit^{®} L100-55 | 99.6 | 0.065 | 0.143 |
| | | (1.582) | |
| Ranolazine/ HPMC Phthalat HP-55 | 99.3 | 0.104 | 0.235 |
| | | (3.539) | |
| Ranolazine/ Aquacoat^{®} CPD | 99.3 | 0.560 | 0.779 |
| | | (3.511) | |

The above data clearly demonstrate that the ranolazine/HPMC-AS composition according to the invention has superior storage properties compared with the prior art compositions and ranolazine per se. The ranolazine / HPMC-AS composition exhibits the lowest value of degradation. Since this value is even lower than the degradation value of ranolazine per se, the HPMC-AS seems to protect the API in the ranolazine / HPMC-AS composition from degrading.

Furthermore the important assay-value of the composition according to the present invention is significant higher with regard to ranolazine per se and compositions comprising ranolazine and prior art binders.

## Claims

1. Pharmaceutical composition comprising
(a) ranolazine
(b) hypromellose-acetate-succinate

2. Pharmaceutical composition according claim 1, further comprising (c) a pore-forming substance

3. Pharmaceutical composition according to claim 1, wherein the amount of ranolazine (a) is lessthan 50% by weight, preferably 35 to less than 50% by weight, based upon the total weight of the pharmaceutical composition.

4. Pharmaceutical composition according to claim 1, wherein the amount of hypromellose-acetate-succinate (b) is between 10 and 65% by weight, preferably 15 and 35% by weight, based upon the total weight of the pharmaceutical composition.

5. Pharmaceutical composition according to claim 2, wherein the amount of the pore-forming substance (c) is between 10 and 65% by weight, preferably 15 to 35% by weight, based upon the total weight of the pharmaceutical composition.

6. Pharmaceutical composition according to claim 2, wherein the pore-forming substance (c) has a water-solubility at 25 °C from 30 g/l to 3000 g/l, preferably from 150 g/l to 300 g/l.

7. Pharmaceutical composition according to claim 2, wherein the pore-forming substance (c) is sugar alcohol, preferably mannitol.

8. Pharmaceutical composition according to any one of the previous claims, further comprising at least one further excipient (d) selected from lubricants (d1), disintegrants (d2), surfactants (d3) glidants (d4), anti-sticking agents (d5), plasticizers (d6) and mixtures thereof.

9. Pharmaceutical composition according to any one of the previous claims, wherein the pharmaceutical composition is in the form of a tablet, said tablet preferably having a hardness between 85 and 275 N.

10. Pharmaceutical composition according to claim 9, wherein the tablet is film-coated with a coating (e).

11. Pharmaceutical composition according to claim 10, wherein the coating (e) comprises
(e1) film-forming agent,
(e2) surfactant,
(e3) lubricant,
(e4) glidant,
(e5) pigments, and/or
(e6) optionally water.

12. Pharmaceutical composition according to any one of the previous claims, wherein the pharmaceutical composition is a modified release pharmaceutical composition.

## Patentansprüche

1. Pharmazeutische Zusammensetzung umfassend
(a) Ranolazin
(b) Hypromelloseacetatsuccinat.

2. Pharmazeutische Zusammensetzung gemäß Anspruch 1 zusätzlich eumfassend (c) eine porenformende Substanz.

3. Pharmazeutische Zusammensetzung gemäß Anspruch 1, wobei der Anteil an Ranolazin (a) weniger als 50 Gew.% beträgt, bevorzugt 35 bis weniger als 50 Gew.%, basierend auf dem Gesamtgewicht der pharmazeutischen Zusammensetzung.

4. Pharmazeutische Zusammensetzung gemäß Anspruch 1, wobei der Anteil an Hypromelloseacetatsuccinat (b) zwischen 10 und 65 Gew.% beträgt, bevorzugt zwischen 15 und 35 Gew.%, basierend auf dem Gesamtgewicht der pharmazeutischen Zusammensetzung.

5. Pharmazeutische Zusammensetzung gemäß Anspruch 2, wobei der Anteil der porenformenden Substanz (c) zwischen 10 und 65 Gew.% beträgt, bevorzugt zwischen 15 und 35 Gew.%, basierend auf dem Gesamtgewicht der pharmazeutischen Zusammensetzung.

6. Pharmazeutische Zusammensetzung gemäß Anspruch 2, wobei die porenformende Substanz (c) eine Wasserlöslichkeit bei 25°C von 30 g/l bis 3000 g/l, bevorzugt von 150 g/l bis 300 g/l, aufweist.

7. Pharmazeutische Zusammensetzung gemäß Anspruch 2, wobei die porenformende Substanz (c) Zuckeralkohol, bevorzugt Mannitol, ist.

8. Pharmazeutische Zusammensetzung gemäß einem der vorangehenden Ansprüche, umfassend zusätzlich mindestens einen weiteren Hilfsstoff (d), ausgewählt aus Schmiermitteln (d1), Sprengmitteln (d2), oberflächenaktiven Substanzen (d3), Fließmitteln (d4), Trennmitteln (d5), Weichmachern (d6) und Mischungen daraus.

9. Pharmazeutische Zusammensetzung gemäß einem der vorangehenden Ansprüche, wobei die pharmazeutische Zusammensetzung in Form einer Tablette vorliegt, wobei die Tablette bevorzugt eine Härte von 85 bis 275 N aufweist.

10. Pharmazeutische Zusammensetzung gemäß Anspruch 9, wobei die Tablette mit einem Überzug (e) befilmt ist.

11. Pharmazeutische Zusammensetzung gemäß Anspruch 10, wobei der Überzug (e)
(e1) Filmbildner,
(e2) oberflächenaktive Substanz,
(e3) Schmiermittel,
(e4) Fließmittel,
(e5) Pigmente, und/oder
(e6) gegebenenfalls Wasser
umfasst.

12. Pharmazeutische Zusammensetzung gemäß einem der vorangehenden Ansprüche, wobei die pharmazeutische Zusammensetzung eine pharmazeutische Zusammensetzung mit modifizierter Freisetzung ist.

## Revendications

1. Composition pharmaceutique comprenant :
(a) de la ranolazine
(b) du succinate d'acétate d'hypromellose.

2. Composition pharmaceutique selon la revendication 1, comprenant en outre :
(c) une substance porogène.

3. Composition pharmaceutique selon la revendication 1, dans laquelle la quantité de ranolazine (a) est inférieure à 50 % en poids, de préférence de 35 à moins de 50 % en poids, sur la base du poids total de la composition pharmaceutique.

4. Composition pharmaceutique selon la revendication 1, dans laquelle la quantité de succinate d'acétate d'hypromellose (b) est comprise entre 10 et 65 % en poids, de préférence entre 15 et 35 % en poids, sur la base du poids total de la composition pharmaceutique.

5. Composition pharmaceutique selon la revendication 2, dans laquelle la quantité de la substance porogène (c) est comprise entre 10 et 65 % en poids, de préférence entre 15 et 35 % en poids, sur la base du poids total de la composition pharmaceutique.

6. Composition pharmaceutique selon la revendication 2, dans laquelle la substance porogène (c) a une solubilité dans l'eau à 25°C de 30 g/L à 3 000 g/L, de préférence de 150 g/L à 300 g/L.

7. Composition pharmaceutique selon la revendication 2, dans laquelle la substance porogène (c) est un polyol, de préférence le mannitol.

8. Composition pharmaceutique selon l'une quelconque des revendications précédentes, comprenant en outre au moins un excipient supplémentaire (d) choisi parmi des lubrifiants (d1), des agents de délitement (d2), des surfactants (d3), des agents de glissement (d4), des agents anti-adhésion (d5), des agents plastifiants (d6) et des mélanges de ceux-ci.

9. Composition pharmaceutique selon l'une quelconque des revendications précédentes, dans laquelle la composition pharmaceutique se trouve sous la forme d'un comprimé, ledit comprimé ayant de préférence une dureté comprise entre 85 et 275 N.

10. Composition pharmaceutique selon la revendication 9, dans laquelle le comprimé est pelliculé avec un enrobage (e).

11. Composition pharmaceutique selon la revendication 10, dans laquelle l'enrobage (e) comprend :
(e1) un agent filmogène,
(e2) un surfactant,
(e3) un lubrifiant,
(e4) un agent de glissement,
(e5) des pigments, et/ou
(e6) facultativement de l'eau.

12. Composition pharmaceutique selon l'une quelconque des revendications précédentes, dans laquelle la composition pharmaceutique est une composition pharmaceutique à libération modifiée.
